# EUROPEAN PATENT APPLICATION

(11) **EP 2 425 812 A1**
(43) Date of publication of application: **07.03.2012**
(21) Application number: 10769788.0
(22) Date of filing: 28.04.2010
(51) Int. Cl.: A61K 8/86, A61K 8/39, A61K 8/60, A61K 8/81, A61Q 5/06

(54) **HAIR-DRESSING COSMETIC**

(30) Priority: 28.04.2009 JP 2009110154
(71) Applicant: Shiseido Company, Ltd., Chuo-ku Tokyo 104-8010 (JP)
(72) Inventor: KURASHIMA, Takumi, Yokohama-shi Kanagawa 224-8558 (JP); SHIMIZU, Hideki, Yokohama-shi Kanagawa 224-8558 (JP); TOYODA, Tomonori, Yokohama-shi Kanagawa 224-8558 (JP); FUJIYAMA, Taizo, Yokohama-shi Kanagawa 224-8558 (JP)
(74) Representative: Uchida, Kenji
(86) International application number: PCT/JP2010/057592
(87) International publication number: WO 2010/126090

(57) **Abstract**

Problem

To provide a hair styling cosmetic composition which is excellent in hair styling property and hair restyling property, even though being water-based and having a low viscosity, and is excellent in non-stickiness, smoothness, and light finish of the hair.

Means for Resolution

A hair styling cosmetic composition comprising (a) (a₁) a polyalkylene glycol polymer or (a₂) a sugar alcohol that is solid at room temperature (25°C), (b) a polyalkylene glycol polymer being liquid at room temperature (25°C), and (c) a film-forming polymer, wherein the ratio of component (a) to component (b) is from 1/0.2 to 1/10 (by mass), the ratio of component (b) to component (c) is from 1/0.1 to 1/1 (by mass), the total content of components (a) to (c) is at least 8% by mass, and the viscosity of the system is at most 10,000 mPa·s (at 25°C with B-type viscometer).

## Description

### TECHNICAL FIELD

The present invention relates to a hair styling cosmetic composition. More precisely, the invention relates to a hair styling cosmetic composition which is excellent in hair styling property and hair restyling property, even though having a low viscosity, and is excellent in non-stickiness, smoothness, and light finish of the hair.

### BACKGROUND ART

Heretofore, in hair styling cosmetic compositions, hair styling resins such as hair-fixing polymer, a film-forming polymer or the like are incorporated for styling. However, hair styling resins are problematic in that they cause stiffness, non-uniformity of films applied on the hair, and reduction in the styling re-tentivity in high-humidity environments. Accordingly, for solving those problems, various countermeasures have been taken.

For example, JP 2007-217314A (Patent Reference 1) describes that a misty powder cosmetic composition containing a polymer compound for hair fixation, a polyalcohol, a monoalcohol and a propellant each in a specific amount is excellent in hair restyling property without stickiness and has a natural glossy appearance.

JP 11-100312A (Patent Reference 2) describes that a hair cosmetic containing a specific low-viscosity polyether compound and a polymer resin compound each in a specific amount has a hair styling property as well as a hair styling retentive property, is free from stickiness and stiffness, and the hair can be restyled by a sweep of the hand even after being blow dried.

JP 3-261713A (Patent Reference 3) describes that a hair cosmetic containing a specific polyoxyalkylene compound and/or polyoxyalkylene alkyl glucoside, a polymer compound for hair fixation, and a high-molecular-weight polyethylene glycol (molecular weight, 6,000 to 30,000) has a hair styling property and give smoothness or evenness.

JP 2002-167317A (Patent Reference 4) describes that a hair cosmetic composition containing an ampholytic polymer, a sugar alcohol, and a sugar alcohol derivative (e.g., polyoxyalkylene adduct to sugar alcohol) has a hair styling property and a hair-set retentive property, and being free from sticky-feeling and stiff-feeling.

JP 2004-505902A (Patent Reference 5) describes that hair care composition containing a specific water-soluble polyalkylene glycol and a film-forming polymer in a specific ratio and further containing a liquid carrier is excellent in a hair restyling property and gives an improved feeling.

However, the above-mentioned patent publications do neither describe nor suggest the purpose or the object of obtaining a hair styling cosmetic composition, especially a water-based low-viscosity hair styling cosmetic composition, capable of sufficiently satisfying both hair styling performance and hair restyling performance; and further in addition thereto, capable of exhibiting non-stickiness, smoothness, and light-finish of the hair. It is especially difficult for a water-based low-viscosity hair styling cosmetic composition to have both a hair-setting property and a hair-arranging property. Accordingly, it has been desired to develop a water-based low-viscosity hair styling cosmetic composition that has both sufficient hair setting property and hair arranging property and has a good feeling in use.

### PRIOR ART REFERENCES

### PATENT REFERENCES

Patent Reference 1: JP 2007-217314A
Patent Reference 2: JP 11-100312A
Patent Reference 3: JP 3-261713A
Patent Reference 4: JP 2002-167317A
Patent Reference 5: JP 2004-505902A

### SUMMARY OF INVENTION

### PROBLEMS THAT INVENTION IS TO SOLVE

The present invention has been made in consideration of the above-mentioned situation, and its object is to provide a hair styling cosmetic composition which is excellent in hair styling performance and hair restyling performance, even though being water-based and having a low viscosity, and is excellent in non-stickiness, smoothness, and light finish of the hair.

### MEANS FOR SOLVING THE PROBLEMS

In order to solve the above-mentioned problems, the invention provides a hair styling cosmetic composition that comprises (a) (a₁) a polyalkylene glycol polymer or (a₂) a sugar alcohol that is solid at room temperature (25°C), (b) a polyalkylene glycol polymer being liquid at room temperature (25°C), and (c) a film-forming polymer, wherein the ratio of component (a) to component (b) is from 1/0.2 to 1/10 (by mass), the ratio of component (b) to component (c) is from 1/0.1 to 1/1 (by mass), the total content of components (a) to (c) is at least 8% by mass, and the viscosity of the system is at most 10,000 mPa·s (at 25°C with B-type viscometer).

The invention also provides the hair styling cosmetic composition, wherein component (a₁) is a polyethylene glycol having a mass-average molecular weight of from 1,000 to 20,000.

The invention also provides the hair styling cosmetic composition, wherein component (b) is a polyethylene glycol having a mass-average molecular weight of from 200 to 900.

The invention also provides the hair styling cosmetic composition, which has a viscosity of at most 100 mPa·s (at 25°C with B-type viscometer) and is used in the form of a fine mist by spraying the composition.

### ADVANTAGEOUS EFFECTS OF INVENTION

According to the invention, there is provided a water-based hair styling cosmetic composition which is excellent in hair styling property and hair restyling property, even though having a low viscosity, and is excellent in non-stickiness, smoothness, and lightly-finishing of the hair.

### MODE FOR CARRYING OUT THE INVENTION

The invention is described in detail hereinunder. In the following, "POE" means "polyoxyethylene", "POP" means "polyoxypropylene", and "POB" means "polyoxybutylene".

### [Component (a)]

Component (a) is either (a₁) a polyalkylene glycol polymer or (a₂) a sugar alcohol, which is solid at room temperature (25°C) . In the invention, one of component (a₁) and component (a₂) is incorporated as component (a). The invention does not include the embodiment where both component (a₁) and component (a₂) are incorporated.

### <Component (a₁)>

Preferred examples of the polyalkylene glycol polymer that is solid at room temperature (25°C) include an EO polymer composed of ethylene oxide (EO) constitutive units polymerized, a PO polymer composed of propylene oxide (PO) constitutive units polymerized, a BO polymer composed of butylene oxide (BO) constitutive units polymerized, as well as the corresponding copolymers composed of the respective constitutive units copolymerized. Especially preferred ones include an EO polymer, an EO/PO copolymer containing EO constitutive units and PO constitutive units, and an EO/BO copolymer containing EO constitutive units and BO constitutive units. The type of copolymerization is not specifically defined, including any of block copolymerization, graft copolymerization, random copolymerization, etc.

As the EO polymer, preferred is polyethylene glycol (PEG) having a mass-average molecular weight (Mw; hereinafter this may be simply referred to as "molecular weight") of at least 1, 000. Not specifically defined, the uppermost limit of the molecular weight is preferably up to about 20, 000 or so. In concrete terms, exemplary ones include PEG having a molecular weight of 1,000 (hereinafter this may be expressed as "PEG 1,000"), PEG 1,540, PEG 2,000, PEG 4, 000, PEG 6, 000, PEG 8, 000, PEG 10, 000, PEG 11, 000, and PEG 20, 000. Above all, PEG 1, 000 to 10, 000 are more preferred, PEG 1, 000 to 8, 000 are even more preferred, and PEG 1,000 to 6,000 are especially preferred from the viewpoint of the hair styling property and hair restyling property.

Preferred examples of the EO/PO copolymer include EO/PO block copolymers represented by the following formula (I):

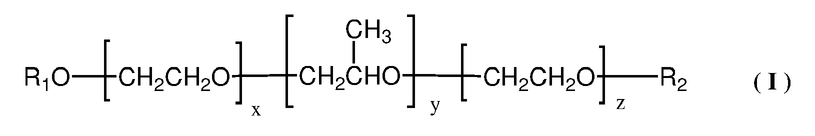

In the above formula (I), the substituents and the symbols have the following meanings.

R₁ and R₂ each independently represent an alkyl group having from 1 to 4 carbon atoms, or a hydrogen atom. The alkyl group having from 1 to 4 carbon atoms includes a methyl group, an ethyl group, an n-propyl group, an isopropyl group, an n-butyl group, a sec-butyl group, and a tert-butyl group. Preferred are a methyl group and an ethyl group. An alkyl group having 5 or more carbon atoms may lower the hydrophilicity of the copolymer and tend to lower the moisturizing feeling.

(x + z) means the added molar number of the EO constitutive units; and y means the added molar number of the PO constitutive units. [(x + z)/(x + z + y)] is from 0.2 to 0.8, preferably from 0.4 to 0.7. When the proportion of the EO constitutive units to the (EO + PO) constitutive units is less than 0.2, then the smoothness tends to be poor; but when more than 0.8, then the stickiness tends to occur.

60 ≤ x + y + z ≤ 100. When (x + y + z) is less than 60, then some types of component (a₁) may be liquid; but when more than 100, then the composition may be sticky and the solubility thereof may be poor.

Specific examples of the EO/PO block copolymer represented by the above formula (I) include POE(35)/POP(40) block copolymer dimethyl ether, and POE(50)/POP(40) block copolymer dimethyl ether.

Preferred examples of the EO/BO copolymer include EO/BO block copolymers represented by the following formula (II):

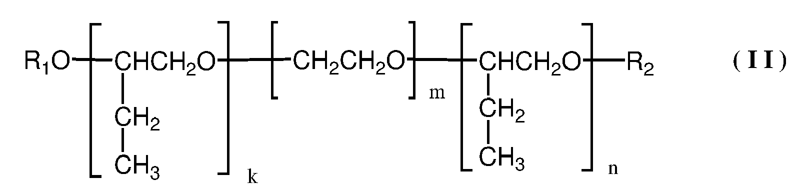

In the formula (II), R₁ and R₂ have the same meanings as in the above formula (I).

m means the added molar number of the EO constitutive units; and (k + n) means the added molar number of the BO constitutive units. [m/(m + k + n)] is from 0.2 to 0.9, preferably from 0.4 to 0.9. When the proportion of the EO constitutive units to the (EO + BO) constitutive units is less than 0.2, then the smoothness tends to be poor; but when more than 0.9, then the sticky-feeling tends to occur.

Preferably, 60 ≤ m + k + n ≤ 100 or so. When (m + k + n) is less than 60, then some types of component (a₁) may be liquid; but when more than 100, then the sticky-feeling tends to occur and the solubility may be poor.

Specific examples of the EO/BO block copolymer represented by the above formula (II) include POE(52)/POB(32) block copolymer dimethyl ether, and POE (73) /POB (11) block copolymer dimethyl ether.

### <Component (a₂)>

The sugar alcohol being solid at room temperature (25°C) is a polyalcohol to be obtained through reduction of the carbonyl group of sugar. Concretely, exemplary sugar alcohol includes maltitol ("Malbit"; by B Food Science Co., Ltd.), sorbitol ("Sorbitol C"; by B Food Science Co., Ltd.), ribitol, mannitol, arabitol, galactitol, xylitol, erythritol, and inositol. Above all, preferred are sorbitol and maltitol from the viewpoint of non-stickiness and non-stiffness.

In the hair styling cosmetic composition of the invention, component (a) that comprises either component (a₁) or component (a₂) mainly contributes to the hair setting property, the hair arranging property, light finish of the hair, and the hair-pliability property. Component (a) is solid at room temperature, and therefore, after the application of the hair styling cosmetic composition on the hair, followed by being volatilized away the solvent from the applied composition, the solid component (a) can remain as widely covering on the hair, and efficacy of component (a) sustain effectively. As described below, the hair styling cosmetic composition of the invention is water-based and has a low viscosity, and hence, the ingredients therein can be widely and evenly applied on the hair.

The amount of component (a) to be used in the hair styling cosmetic composition of the invention is preferably from 0.1 to 20% by mass, more preferably from 3 to 8% by mass. When the amount is less than 0.1% by mass, then component (a) could not sufficiently exert its property; but on the other hand, even though the amount is more than 20% by mass, not only there is no expecting a further improvement in efficacy that is commensurate with the increased amount of its use, but also the viscosity of the composition may increase, which is therefore unfavorable in point of the oily feeling, stickiness, and heavy finish of the hair. Component (a₁) or component (a₂) each may be used either alone or in combination.

### [Component (b)]

Preferred examples of the polyalkylene glycol polymer that is liquid at room temperature (25°C) include an EO polymer composed of EO constitutive units polymerized, a PO polymer composed of PO constitutive units polymerized, a BO polymer composed of BO constitutive units polymerized, as well as the corresponding copolymers composed of the respective constitutive units copolymerized. Especially preferred ones include an EO polymer, an EO/PO copolymer containing EO constitutive units and PO constitutive units, an EO/BO copolymer containing EO constitutive units and BO constitutive units. The type of copolymerization is not specifically defined, including any of block copolymerization, graft copolymerization, random copolymerization.

As the EO polymer, preferred ones include polyethylene glycol (PEG) having a molecular weight of at most 900 from the viewpoint of light finish of hair, smoothness, and non-stickiness; and more preferred ones include polyethylene glycol having a molecular weight of at most 600. Not specifically defined, the lowermost limit of the molecular weight is preferably at least about 200 or so, more preferably at least about 300 or so. Concretely, exemplary are PEG200, PEG300, PEGH400, and PEG600.

Preferred examples of the EO/PO copolymer include random copolymers represented by the following formula (III):

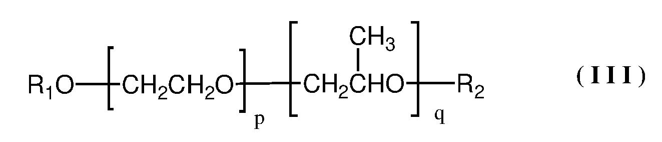

In the above formula (III), the ratio of p (= added molar number of the EO constitutive units) to q (= added molar number of the PO constitutive units), p/q is from 10 to 0.5, preferably from 5 to 0.8. 2 ≤ p + q ≤ 100.

Specific examples of the copolymer represented by the above formula (III) include POE(9)/POP(2) random copolymer dimethyl ether, POE(14)/POP(7) random copolymer dimethyl ether, POE(36)/POP(41) random copolymer dimethyl ether, and POE(55)/POP(28) random copolymer dimethyl ether.

In the hair styling cosmetic composition of the invention, component (b) mainly contributes to the hair restyling property, non-stickiness and smoothness. Component (b) may be used either alone or in combination. In the invention, especially preferred is use of polyethylene glycol having a molecular weight of from 200 to 900.

The amount of component (b) to be used in the hair styling cosmetic composition of the invention is preferably from 0.1 to 30% by mass, more preferably from 5 to 15% by mass. When the amount is less than 0.1% by mass, then component (b) could not sufficiently exert its property; but on the other hand, even though the amount is more than 30% by mass, not only there is no expecting a further improvement in efficacy that is commensurate with the increased amount of its use, but also the viscosity of the composition may increase, which is therefore unfavorable in point of stickiness, and heavy finish of the hair.

### [Component (c)]

Not specifically defined, the film-forming polymer may be any film-forming polymer heretofore used in hair styling cosmetic compositions such as hair styling agents. In the invention, preferred ones are acrylic, vinylic or urethanic film-forming polymers from the viewpoint of the hair restyling property.

### <Acrylic or Vinylic Film-Forming Polymer>

Exemplary anionic polymers include alkyl acrylate/diacetone acrylamide copolymer [Plas Cize L-53P, Plas Cize L-9909B, Plas Cize L-9948B, etc. (all by Goo Chemical Co., Ltd.)], alkyl acrylate/octylacrylamide copolymer [Dermacryl 79 (by AkzoNobel)], polyethylene glycol/polypropylene glycol-25/dimethicone acrylate copolymer [Rubiflex SILK (by BASF)], acrylic acid/acrylic acid amide/ethyl acrylate copolymer [Ultrahold 8, Ultrahold Strong (both by BASF)], and alkyl acrylate copolymer [Aniset NF-1000, Aniset HS-3000, etc. (all by Osaka Organic Chemical Industry Ltd.)].

Exemplary ampholytic polymers include acrylic acid octylamide/hydroxypropylpropyl acrylate/butylaminoethyl methacrylate copolymer [AMPHOMER SH30, AMPHOMER LV-71 (both by AkzoNobel)], methacryloyloxyethylcarboxybetaine/alkyl methacrylate copolymer [Yukaformer R205, Yukaformer 301, Yukaformer SM, Yukaformer 104D, etc. (all by Mitsubishi Chemical Corp.), RAM Resin-1000, RAM Resin-2000, RAM Resin-3000, RAM Resin-4000 (all by Osaka Organic Chemical Industry Ltd.)], dimethyldiallylammonium chloride/acrylic acid copolymer [Merquat 280, Merquat 295 (both by Nalco)], and dimethyldiallylammonium chloride/acrylamide/acrylic acid copolymer [Merquat Plus 3330, Merquat Plus 3331 (both by Nalco)].

Exemplary cationic polymers include vinylpyrrolidone/dimethylaminoethyl methacrylate copolymer diethyl sulfate salt [H.C. Polymer 1S(M), H.C. Polymer 2 (both by Osaka Organic Chemical Industry Ltd.), Gafquat 755N (by ISP)], vinylpyrrolidone/dimethylaminopropylmethacrylamide/lauryldimethylaminopropy lmethacrylamide copolymer [Styleze W-20 (by ISP)], vinylpyrrolidone/N,N-dimethylaminoethyl methacrylate/alkyl acrylate/tripropylene glycol diacrylate copolymer [Cosquat GA467, Cosquat GA468 (both by Osaka Organic Chemical Industry Ltd.)], polydimethylmethylenepiperidinium chloride [Merquat 100 (by Nalco)], dimethyldiallylammonium chloride/acrylamide copolymer [Merquat 550 (by Nalco)], and trimethylaminopropylacrylamide chloride/dimethylacrylamide copolymer.

Exemplary nonionic polymers include polyvinylpyrrolidone [Luviskol K17, Luviskol K30, Luviskol K90 (all by BASF), PVP K (by ISP)], vinylpyrrolidone/vinyl acetate copolymer [PVP/VA S-630, PVP/VA E-735, PVP/VA E-335 (all by ISP), Luviskol VA73W, Luviskol 37E (both by BASF), PVA-6450 (by Osaka Organic Chemical Industry Ltd.)], vinyl methyl ether/alkyl maleate copolymer [Gauntlets A-425, Gauntlets ES-225, Gauntlets ES-335 (all by ISP)], and vinylpyrrolidone/methacrylamide/vinylimidazole copolymer [Luviset Clear (by BASF)].

### <Urethanic Film-Forming Polymer>

Exemplary urethanic film-forming polymers include mentioned silicone/polyether polyurethane resin [Yodosol PUD, by AkzoNobel], "Luviset P.U.R." (by BASF), and silylated urethanic polymer described in JP 2006-213706A. Exemplary acrylic-urethanic film forming polymers include "DynamX" (by AkzoNovel).

In the hair styling cosmetic composition of the present invention, component (c) mainly contributes to the hair setting property, the hair restyling property, and light finish of the hair. Component (c) may be used either alone or in combination.

The amount of component (c) to be used in the hair styling cosmetic composition of the invention is preferably from 0.1 to 15% by mass, more preferably from 2 to 6% by mass. When the amount is less than 0.1% by mass, then component (c) could not sufficiently exert its property; but on the other hand, even though the amount is more than 15% by mass, not only there is no expecting a further improvement in efficacy that is commensurate with the increased amount of its use, but also the viscosity of the composition may increase, which is therefore unfavorable in point of stiffness of the hair.

The hair styling cosmetic composition of the invention contains the above-mentioned components (a) to (c) as the indispensable ingredients therein. The preferred amount of components (a) to (c) is as described above; and in the invention, for the purpose of sufficiently exhibiting the advantageous effects of the invention, the total amount of components (a) to (c) is at least 8% by mass, preferably from 10 to 30% by mass. When the total amount of components (a) to (c) is less than 8% by mass, then the composition could not exhibit sufficient hair styling performance and hair restyling performance.

In the present invention, the ratio of component (a) to component (b) is from 1/0.2 to 1/10 (by mass), preferably from 1/0.3 to 1/3. When the proportion of component (a) is larger than the above range, then the treated hair may be more sticky and more stiff and could not be smooth, and in addition, the hair styling property and the hair restyling property of the composition would not be sufficient; on the other hand, when the proportion of component (a) is smaller than the above range, then the proportion of component (b) relatively increase and the composition could not secure the hair styling property and would be insufficient in point of the hair restyling property and the light finish of the hair.

The ratio of component (b) to component (c) is from 1/0.1 to 1/1 (by mass), preferably from 1/0.3 to 1/0.7. When the proportion of component (c) is larger than the range, then it would cause stiffness and stickiness of the hair and in addition, the composition could not have a hair restyling property; but on the other hand, when the proportion of component (c) is smaller than the above range, then the hair styling property and the hair restyling property of the composition would be insufficient.

In addition to the above-mentioned components (a) to (c), the hair styling cosmetic composition of the invention may contain any other ingredient within a range not detracting from the advantageous effects of the invention. Such ingredient includes polysaccharide-type polymers.

Exemplary polysaccharide-type polymers include gum arabic, glucan, succinoglycan, carrageenan, karaya gum, tragacanth gum, guar gum, locust bean gum, galactomannan gum, xanthane gum, starch, carob gum, quince seed (Cydonia oblonga), casein, dextrin, gelatin, sodium pectate, sodium alginate, methyl cellulose, ethyl cellulose, carboxymethyl cellulose, hydroxyethyl cellulose, hydroxypropyl cellulose, crystalline cellulose, O-[2-hydroxy-3-(trimethylammonio)propyl]hydroxyethyl cellulose chloride, O-[2-hydroxy-3-(trimethylammonio)propyl]guar gum chloride, O-[2-hydroxy-3-(trimethylammonio)propyl]locust bean gum chloride, and hydroxypropyltrimethylammonium chloride starch. Adding such a polysaccharide-type polymer can more effectively control the viscosity, the hair styling property, and light finish of the hair.

In the case where the polysaccharide-type polymer is incorporated, its amount to be added may be suitably controlled in consideration of the viscosity control, the hair styling property, and light finish of the hair. Preferably, the polymer amount is from 0.01 to 1% by mass, more preferably from 0.05 to 0.5% by mass in the hair styling cosmetic composition.

The viscosity of the system of the hair styling cosmetic composition of the invention is at most 10,000 mPa·s (at 25°C with B-type viscometer), preferably at most 1, 000 mPa·s. In particular, in the case where the hair styling cosmetic composition of the invention is used by spraying it in the form of a mist or the like in use thereof, the viscosity is preferably at most 100 mPa·s. Not specifically defined, the lowermost limit of the viscosity is preferably at least 8 mPa·s or so from the viewpoint of the feeling in use.

The hair styling cosmetic composition of the invention is one in which components (a) to (c) and any other optional ingredient are dissolved in a water-based solvent (e.g., water, alcoholic solvents such as ethanol, etc.; or their mixed solvents) . The hair styling cosmetic composition of the invention is water-based and has a low viscosity, and its viscosity may be controlled, for example, by controlling the degree of polymerization of the copolymer to be incorporated, by increasing or decreasing the amount of the polymers to be added, and by controlling the amount of the water-based solvent to be incorporated.

Heretofore, water-based low-viscosity hair styling cosmetic compositions could hardly exhibit sufficient hair styling performance and hair restyling performance. Though having a low viscosity, the hair styling cosmetic composition of the present invention has secured sufficient hair styling performance and hair restyling performance. In addition, as being water-based and having a low viscosity, the hair styling cosmetic composition can be stably sprayed as a mist without any trouble of nozzle clogging, even when it is in the form of a hair mist or the like preparation to be sprayed in use thereof.

In addition to the above-mentioned ingredients, any other ingredient generally used in cosmetics, drugs or the like may be optionally incorporated in the hair styling cosmetic composition of the invention within a range not detracting from the advantageous effects of the invention. The ingredient includes powder, liquid oil and fat, wax, hydrocarbon oil, higher fatty acid, higher alcohol, ester oil, silicone oil, anionic surfactant, cationic surfactant, ampholytic surfactant, nonionic surfactant, UV absorbent, polyhydric alcohol, metal ion sequestrant, sugar, amino acid, organic amine, polymer emulsion, pH controlling agent, skin nutrient, vitamin, antioxidant, antioxidation promoter, and fragrance. These ingredients may be optionally suitably incorporated, and the hair styling cosmetic composition of the invention can be produced according to the intended preparation form thereof in an ordinary manner.

The hair styling cosmetic composition of the invention may be in any form of a dissolution system, an emulsion system, a powder dispersion system, an oil-water two-layer system, or an oil-water-powder three-layer system. Preferred use embodiments of the hair styling cosmetic composition of the invention include an aerosol-type hair spray, a non-aerosol-type hair spray, a hair mist, a set lotion, a hair-styling gel, and a hair liquid.

As being water-based and having a low viscosity, the hair styling cosmetic composition of the invention exhibits excellent hair styling performance and hair restyling performance and therefore, even though in the form of a hair spray, a hair mist or the like that is used by spraying, the hair styling cosmetic composition can be widely and evenly applied onto hair, without clogging the spray nozzle of the spray container. An aerosol-type cosmetic product is generally charged in a spray container along with a propellant therein. As the propellant, herein usable is any and every propellant known in the field of aerosols, such as liquefied gas of propane, butane, pentane, dimethyl ether or the like, nitrogen, or compressed gas such as compressed air, etc. The amount of the propellant to be incorporated is preferably from 30 to 60% by mass relative to 100% by mass of the hair styling cosmetic composition (stock solution).

### EXAMPLES

The invention is described in detail with reference to the following Examples, but not limited thereto. Unless otherwise specifically indicated, the incorporated is expressed by % by mass (as actual content).

First described are the evaluation methods used in these Examples.

### [Viscosity]

A sample (100 to 200 mL) was put into a BL-type viscometer (rotor No. 2, number of revolution 60 rpm, 25 ± 2°C), and the sample viscosity was measured after the lapse of 1 minute from the start of the rotor rotation.

### [Hair styling property]

0.5 g of a sample was applied onto a bundle of black virgin hair (length 20 cm, mass 4 g), then rubbed into the hair with fingers, and thereafter evaluated according to the sensory test conducted by women expert panelists (10 women) regarding the easiness in hair styling.

### [Hair restyling property]

0.5 g of a sample was applied onto a bundle of black virgin hair (length 20 cm, mass 4 g), then dried at room temperature for 1 hour, and evaluated according to the sensory test conducted by women expert panelists (10 women) regarding the easiness in arranging the hair style by pinching, twisting and moving the hair.

### [Non-stickiness]

0.5 g of a sample was applied onto a bundle of black virgin hair (length 20 cm, mass 4 g), then rubbed into the hair with fingers, and evaluated according to the sensory test conducted by women expert panelists (10 women) regarding the non-stickiness of the hair.

### [Smoothness]

0.5 g of a sample was applied onto a bundle of black virgin hair (length 20 cm, mass 4 g), then rubbed into the hair with fingers and finished styling, and thereafter evaluated according to the sensory test conducted by women expert panelists (10 women) regarding the smoothness of the hair surface.

### [Light Finish of Hair]

0.5 g of a sample was applied onto a bundle of black virgin hair (length 20 cm, mass 4 g), then rubbed into the hair with fingers and finished styling, and thereafter evaluated according to the sensory test conducted by women expert panelists (10 women) regarding the light finish of the hair.

### <Rating Point>

5: Excellent
4: Good
3: Average (neither good or not good)
2: Somewhat not good
1: Not good

### <Evaluation Standard>

Θ: At least 40 points as the total rating point.
○: From 30 points to less than 40 points as the total rating point.
Δ: From 20 points to less than 30 points as the total rating point
×: Less than 20 points as the total rating point.

### (Comparative Examples 1 to 12, Examples 1 to 3)

The samples shown in Tables 1 and 2 below were evaluated in point of the hair styling property, the hair restyling property, the non-stickiness, the smoothness and the light finish of the hair, according to the above-mentioned evaluation methods. The results are shown in Tables 1 and 2. In Tables 1 and 2, the following commercial products were used for the following ingredients.
Maltitol^{(*)}: "Malbit" (by B Food Science Co., Ltd.).
Methacryloyloxyethylcarboxybetaine/alkyl methacrylate copolymer^{(**)}: "Yukaformer 301" (30% solution, by Mitsubishi Chemical Corp.).

**[Table 1]**

| Constitulive Ingredients | | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|
| Ion-Exchanged Water | | bal. | bal. | bal. | bal. | bal. | bal. | bal. | bal. |
| Ethanol | | 40 | 40 | 40 | 40 | 40 | 40 | 40 | 40 |
| Polyethylene Glycol (molecular weight 1540) | | - | 5 | 5 | 5 | 5 | - | - | 12 |
| Polyethylene Glycol (molecular weight 10000) | | - | - | - | - | - | - | - | - |
| Maltitol (*) | | - | - | - | - | - | 5 | 5 | - |
| 1,3-Butylene Glycol | | - | - | - | - | 7 | - | 7 | - |
| Polyoxypropylene(40) Butyl Ethel | | - | - | - | 7 | - | 7 | - | - |
| Polyethylene Glycol (molecular weight 400) | | 7 | - | 7 | - | - | - | - | 2 |
| Methaayloyloxyethylcarboxybetaine/Alkyl Methacrylate Copolymer (**) (amount, as actual content) | | 2.1 | 2.1 | - | 2.1 | 2.1 | 2.1 | 2.1 | 2.1 |
| Fragrance | | 0.2 | 0.2 | 0.2 | 0.2 | 0.2 | 0.2 | 0.2 | 0.2 |
| | Viscosity (mPa·s/25°C) | 10 | 11 | 11 | 15 | 13 | 11 | 13 | 20 |
| | Hair styling property | Δ | Δ | × | ○ | Δ | ○ | Δ | ○ |
| | Hair restyling property after 1 hour | × | Δ | Δ | Δ | × | Δ | × | × |
| | Non-sUddness | ○ | × | ○ | Δ | Δ | × | Δ | × |
| | Smoothness | ○ | × | ○ | ○ | Δ | Δ | Δ | × |
| | LishtFinshofHair | ○ | Δ | ○ | × | ○ | × | Δ | Δ |

**[Table 2]**

| Constitutive Ingredients | | Comp. Ex. 9 | Comp. Ex. 10 | Comp. Ex. 11 | Comp. Ex. 12 | Ex. 1 | Ex. 2 | Ex. 3 |
|---|---|---|---|---|---|---|---|---|
| Ion-Exchanged Water | | bal. | bal. | bal. | bal. | bal. | bal. | bal. |
| Ethanol | | 40 | 40 | 40 | 40 | 40 | 40 | 40 |
| Polyethylene Glycol (molecular weight 1540) | | 1 | 2.5 | 5 | 5 | 5 | - | - |
| Polyethylene Glycol (molecular weight 10000) | | - | - | - | - | - | 5 | - |
| Maltitol (*) | | - | - | - | - | - | - | 5 |
| 1,3.Butylene Glycol | | - | - | - | - | - | - | - |
| Polyoxypropylene(40) Butyl Ethel | | - | - | - | - | - | - | - |
| Polyethylene Glycol (molecular weight 400) | | 12 | 3.5 | 9.5 | 4.5 | 7 | 7 | 7 |
| Methacryloyloxyethylcarboxybetaine/Alkyl Methacrylate Copolymer (**) (amount, as actual content) | | 2.1 | 1.5 | 0.6 | 5.1 | 2.1 | 2.1 | 2.1 |
| Fragrance | | 0.2 | 0.2 | 0.2 | 0.2 | 0.2 | 0.2 | 0.2 |
| | Viscosity (mPa·s/25°C) | 15 | 12 | 13 | 16 | 13 | 16 | 12 |
| | Hair styling property | × | Δ | Δ | ○ | ○ | Θ | Θ |
| | Hair restyling property after 1 hour | Δ | Δ | Δ | × | ○ | ○ | ○ |
| | Non-stiokiness | ○ | ○ | ○ | Δ | ○ | ○ | Δ |
| | Smoothness | ○ | Δ | ○ | Δ | ○ | ○ | ○ |
| | Light Finish of Hair | ○ | ○ | ○ | ○ | ○ | △ | ○ |

As obvious from the results in Tables 1 and 2, the hair styling cosmetic composition of the invention can exhibit both the effects of hair styling performance and hair restyling performance excellently and with a good balance therebetween, though having a low viscosity, and additionally can have the effects of non-stickiness, smoothness and light finish of the hair. On the other hand, the hair styling cosmetic composition beyond the scope of the invention could not exhibit all the advantageous effects of the invention. AS is clearly shown from the comparison of Examples 1 - 3 with Comparative Examples 5 - 7, the advantages of the present invention could not be achieved when component (b) is replaced by any other ingredient such as moisturizer, polyhydric alcohol, or oil for hair styling.

Next shown are formulation examples hereinunder.

### (Example 4: Mist-type hair styling agent)

| | (Constitutive Ingredients) | (% by mass) |
|---|---|---|
| (1) | Ion-exchanged water | bal. |
| (2) | Ethanol | 40 |
| (3) | Polyethylene glycol (molecular weight 1540) | 4 |
| (4) | Polyethylene glycol (molecular weight 6000) | 1 |
| (5) | Polyethylene glycol (molecular weight 400) | 10 |
| (6) | 1,3-Butylene glycol | 1 |
| (7) | Vinylpyrrolidone/vinyl acetate copolymer | 3 |
| | (PVA/VA S-630, by ISP) | |
| (8) | (Dimethylacrylamide/acryloyldimethyltaurine Na) cross polymer | 0.05 |
| | (SUpolymer G-1, by Toho Chemical Industry) | |
| (9) | Citric acid (edible) | q.s. |
| (10) | Sodium citrate | q.s. |
| (11) | Fragrance | 0.1 |
| (12) | Hydrolyzed wheat protein | 0.01 |

### Production Method:

(6), (9) and (10) are dissolved in (1) to prepare an aqueous phase part. (7), (8), (11) and (12) are dissolved in (2) to prepare an alcohol part. Subsequently, the alcohol part, (5) and heated and melted (3) and (4) are sequentially put into the aqueous phase part and mixed with stirring to prepare a transparent, mist-type hair styling agent.

### (Example 5: Mist-type hair styling agent)

| | (Constitutive Ingredients) | (% by mass) |
|---|---|---|
| (1) | Ion-exchanged water | bal. |
| (2) | Ethanol | 5 |
| (3) | Maltitol | 3 |
| (4) | Polyethylene glycol (molecular weight 600) | 3 |
| (5) | Polyethylene glycol (molecular weight 300) | 5 |
| (6) | Diglycerin | 2 |
| (7) | Polydimethylmethylenepiperidinium chloride | 3 |
| | (Marcoat 100, by Nalco) | |
| (8) | Silicone/polyether-type polyurethane resin | 1 |
| | (Yodosol PUD, by AkzoNobel) | |
| (9) | Vinylpyrrolidone/N,N'-dimethylaminoethyl methacrylate/stearyl acrylate/tripropylene glycol diacrylate copolymer (CG Polymer (D), by Osaka Organic Chemical Industry) | 0.1 |
| (10) | Phenoxyethanol | 0.5 |
| (11) | Fragrance | 0.1 |
| (12) | Soybean lecithin | 0.1 |

### Production Method:

(3), (6) and (9) are dissolved in (1) to prepare an aqueous phase part. (7), (8), (10), (11) and (12) are dissolved in (2) to prepare an alcohol part. Subsequently, the alcohol part, (4) and (5) are sequentially put into the aqueous phase part and mixed with stirring to prepare a transparent, mist-type hair styling agent.

### (Example 6: Gel-type hair styling agent)

| | (Constitutive Ingredients) | (% by mass) |
|---|---|---|
| (1) | Ion-exchanged water | bal. |
| (2) | Ethanol | 40 |
| (3) | Sorbitol | 5 |
| (4) | Maltitol | 5 |
| (5) | Polyethylene glycol (molecular weight 300) | 5 |
| (6) | Vinylpyrrolidone/vinyl acetate copolymer | 1 |
| | (PVP/VA S-630, by ISP) | |
| (7) | Alkyl acrylate/diacetonacrylamide copolymer | 3 |
| | (Plas Cize L-9909B, by Goo Chemical Industry) | |
| (8) | Carboxyvinyl polymer (Hibiswako 105, by Wako Pure Chemical Industries) | 0.2 |
| (9) | POE decyltetradecyl ether/hexamethylene diisocyanate/polyethylene glycol 11000 copolymer (Adekanol GT-700, by Asahi Denka) | 0.2 |
| (10) | Caustic potash | 0.1 |
| (11) | Fragrance | 0.1 |
| (12) | Hydroxyethyl urea | 0.1 |

### Production Method:

(3), (4), (5), (8), (9) and (10) are dissolved in (1) to prepare an aqueous phase part. (6), (11) and (12) are dissolved in (2) to prepare an alcohol part. Subsequently, the aqueous phase part and the alcohol parts are mixed with stirring to prepare a transparent gel-type hair styling agent.

### (Example 7: Aerosol spray-type hair styling agent)

| | (Constitutive Ingredients) | (% by mass) |
|---|---|---|
| (1) | Ion-exchanged water | bal. |
| (2) | Ethanol | 50 |
| (3) | Polyethylene glycol (molecular weight 1540) | 8 |
| (4) | POE(35)/POP(40) block copolymer dimethyl ether | 2 |
| | [= copolymer of the above-mentioned formula (I)] | |
| (5) | Polyethylene glycol (molecular weight 400) | 5 |
| (6) | Methacryloyloxyethylcarboxybetaine/alkyl methacrylate copolymer | 6 |
| | (Yukaformer 301, by Mitsubishi Chemical) | |
| (7) | Fragrance | 0.1 |
| (8) | L-menthol | 0.1 |

### Production Method:

(6), (7) and (8) are dissolved in (2) to prepare an alcohol part. The alcohol part, (5), and heated and melted (3) and (4) are sequentially put into (1) and mixed with stirring to prepare a stock liquid. 60% of the stock liquid and 40% of diethyl ether are charged in an aerosol can and mixed therein to prepare an aerosol spray.

### (Example 8: Cream-type hair styling agent)

| | (Constitutive Ingredients) | (% by mass) |
|---|---|---|
| (1) | Ion-exchanged water | bal. |
| (2) | Polyethylene glycol (molecular weight 6000) | 3 |
| (3) | POE(73)/POB(11) block copolymer dimethyl ether [= copolymer of the above-mentioned formula (II)] | 2 |
| (4) | Polyethylene glycol (molecular weight 300) | 3 |
| (5) | Propylene glycol | 3 |
| (6) | Silicone/polyether-type polyurethane resin | 2 |
| | (Yodosol PUD, by AkzoNobel) | |
| (7) | Acrylic acid/alkyl methacrylate copolymer | 0.1 |
| | (PEMULEN TR-2, by Lubrizol Advanced Materials, Inc.) | |
| (8) | Liquid paraffin | 10 |
| (9) | Vaseline | 5 |
| (10) | Microcrystalline wax | 5 |
| (11) | Stearyl alcohol | 2 |
| (12) | Carnauba wax | 3 |
| (13) | Isostearic acid | 1 |
| (14) | Stearic acid | 2 |
| (15) | Pentaerythritol tetra-2-ethylhexanoate | 3 |
| (16) | Polyoxyethylene glyceryl isostearate | 1 |
| (17) | Self-emulsifying glycerin monostearate | 2 |
| (18) | Kaolin | 5 |
| (19) | Bentonite | 1 |
| (20) | Silicic anhydride | 2 |
| (21) | Triethanolamine | q.s. |
| (22) | Phenoxyethanol | 0.5 |
| (23) | Fragrance | 0.1 |
| (24) | Wine extract | 0.001 |

### Production Method:

(5) and (7) are dissolved in (1) to prepare an aqueous phase part. Subsequently, (8) to (19) are mixed and melted under heat, and (21) to (24) are added thereto and melted to prepare an oily phase part. The oily phase part and (20) are mixed with the aqueous phase part and emulsified using a homomixer, and then (4), (6), and heated and melted (2) and (3) are sequentially added thereto and mixed with stirring to prepare a cream-type hair styling agent.

### INDUSTRIAL APPLICABILITY

The hair styling cosmetic composition of the invention is excellent in hair styling property and hair restyling property, even though being water-based and having a low viscosity, and is excellent in non-stickiness, smoothness, and light finish of the hair.

## Claims

1. A hair styling cosmetic composition comprising:
(a) (a₁) a polyalkylene glycol polymer or (a₂) a sugar alcohol that is solid at room temperature (25°C),
(b) a polyalkylene glycol polymer being liquid at room temperature (25°C), and
(c) a film-forming polymer,
wherein the ratio of component (a) to component (b) is from 1/0.2 to 1/10 (by mass), the ratio of component (b) to component (c) is from 1/0.1 to 1/1 (by mass), the total content of components (a) to (c) is at least 8% by mass, and the viscosity of the system is at most 10,000 mPa·s (at 25°C with B-type viscometer).

2. The hair styling cosmetic composition as claimed in claim 1, wherein component (a) is a polyethylene glycol having a mass-average molecular weight of from 1,000 to 20,000.

3. The hair styling cosmetic composition as claimed in claim 1 or 2, wherein component (b) is a polyethylene glycol having a mass-average molecular weight of from 200 to 900.

4. The hair styling cosmetic composition as claimed in claim 1, which has a viscosity of at most 100 mPa·s (at 25°C with B-type viscometer) and is used in the form of a fine mist by spraying the composition.
